# EUROPEAN PATENT SPECIFICATION

(11) **EP 3 107 526 B1**
(45) Date of publication and mention of the grant of the patent: **29.07.2020**
(21) Application number: 15752673.2
(22) Date of filing: 18.02.2015
(51) Int. Cl.: A61K 8/891, A61K 8/89, A61K 8/92, A61Q 17/04, A61K 8/37, A61K 8/895, A61K 8/11, A61K 8/31

(54) **TOPICAL ANHYDROUS FILL FORMULATION FOR ULTRAVIOLET FILTERS**
TOPISCHE WASSERFREIE FÜLLSTOFFFORMULIERUNG FÜR UV-FILTER
FORMULATION ANHYDRE TOPIQUE DE REMPLISSAGE POUR FILTRES ANTI-ULTRAVIOLETS

(30) Priority: 18.02.2014 US 201461941200 P
(43) Date of publication of application: 28.12.2016
(73) Proprietor: R.P. Scherer Technologies, LLC, Las Vegas, Nevada 89119 (US)
(72) Inventor: MARIGLIANO, Ilaria, I-00175 Rome (IT); PIRONI, Laura, I-00144 Rome (IT)
(74) Representative: De Vries & Metman
(86) International application number: PCT/US2015/016267
(87) International publication number: WO 2015/126874

(56) References cited:
- WO-A1-2012/143344
- JP-A- 2003 063 927
- US-A- 5 082 661
- US-A1- 2004 005 279
- US-A1- 2004 228 821
- US-A1- 2010 010 091
- US-A1- 2010 330 011
- US-A1- 2011 200 543
- US-A1- 2012 189 676
- US-A1- 2012 321 576
- US-A1- 2013 071 341
- US-A1- 2013 230 474
- US-B1- 6 248 339
- DATABASE GNPD [Online] MINTEL; September 2009 (2009-09), "Unversal UV shield SPF 30", XP002770531, Database accession no. 1159837
- DATABASE GNPD MINTEL; "Natural Sunblock SPF 50", XP002770532, Database accession no. 1746261
- DATABASE GNPD [Online] MINTEL; April 2013 (2013-04), "Résistantiel UV AGXpert SPF 50/PA++++", XP002770533, Database accession no. 2056785
- DATABASE GNPD [Online] MINTEL; February 2005 (2005-02), "Quickies Pocket Packs Photo Age Protection Gel", XP002770534, Database accession no. 10207971
- DATABASE GNPD [Online] MINTEL; February 2005 (2005-02), "Everyday Protective Base", XP002770535, Database accession no. 336297
- DATABASE GNPD [Online] MINTEL; August 2004 (2004-08), "Glymed Plus - Body Sun Gel", XP002770536, Database accession no. 10184368
- DATABASE GNPD [Online] MINTEL; August 2003 (2003-08), "Krem Ochronny do Cery Fotowrazliwej Body Cream", XP002770537, Database accession no. 223494
- DATABASE GNPD [Online] MINTEL; 3 November 2000 (2000-11-03), anonymous: "Perfect Eye Care", XP055604167, retrieved from www.gnpd.com Database accession no. 77895
- DATABASE GNPD [Online] MINTEL; 4 August 2011 (2011-08-04), anonymous: "#3 Ceramide Moisture Care Face Capsules", XP055604196, retrieved from www.gnpd.com Database accession no. 1601720
- DATABASE GNPD [Online] MINTEL; 7 June 2012 (2012-06-07), anonymous: "Wrinkle Lift Capsules", XP055604199, retrieved from www.gnpd.com Database accession no. 1799237

## Description

### BACKGROUND OF THE INVENTION

### 1. Field of the Invention

The invention relates to fill formulations providing a stable anhydrous vehicle for ultraviolet (UV) filters and optionally one or more UV defense boosters. More specifically, the fill formulation provides the ability to include sun protection in skin care formulations that are compatible with capsules, pearls and drops unit dose technologies .

### 2. Description of the Related Technology

There are a lot of theories on aging and some estabilished ones consider not only the chronologic role, which is related to genetic, structural factors as well as as to a series of intrinsic phenomena (Chrono-aging), but also the role of photo induced free radicals, which accelarete and anticipate skin aging (Photo-aging). Photo-aging depends primarly on the degree of sun exposure and skin pigment (photo-type).

It is well known that it is important to protect your skin from the sun every day. Whether spending a day at the beach or running errands, sun protection is essential. You should apply sunscreen every day to all skin that is not covered by clothing. Increasingly, a variety of skincare products have an SPF built in for day to day protection.

It would be of value to have soft gelatin non gelatin based (also known as Vegicaps® capsules or OptiShell™ capsules) capsules encapsulating topical formulations containing UV filters.

Sun protection in the form of a variety of different products is currently in demand in the marketplace. However, such sun protection must deliver a minimum Sun Protection Factor (SPF), typically at least SPF 15 is required. However, many difficulties have been encountered in the formulation of topical products that are compatible with capsule shell technologies such as gelatin based capsules and Vegicaps® or OptiShell™ capsules and which meet actual market requirements for products containing UV filters and UV defense boosters.

US 2012/189676 A1 discloses a composition comprising at least one α-cyanodiphenylacrylate and at least oxyalkylenated organosiloxane emulsifier, and a method for stabilizing a composition by formulating with at least one α-cyanodiphenylacrylate and at least one oxyalkylenated organosiloxane emulsifier. Example 8 of this application is directed to a foundation makeup which comprises less than 22 wt.% of isododecane, 56.77 wt.% silicone elastomers (plus an additional contribution from the dimethicone crosspolymer-3/isododecane mixture which is present in an amount of 22 wt.%), 4.0 wt.% of polyethylene, and UV filter.

U.S. Patent no. 5,082,661 discloses a cosmetic product in the form of a gelatin walled capsule encompassing a cosmetic composition that includes a carrier which is a silicone polymer and an antioxidant. The antioxidant operates to inhibit degradation of the gelatin wall to prevent malodors from being generated. Retinoic acid derivatives such as retinyl palmitate are especially effective as antioxidants.

One challenge is that a drop in viscosity is observed, particularly in anhydrous formulations, when a UV filter is added to the formulation. The drop in viscosity has a negative effect on the texture and skin feeling of the product and an impact on the film forming property of the formulation when applied to the skin. Also, the viscosity of the product influences the physical stability of the formulation, as well as the ability to reproducably encapsulate the formulation. The film-forming and textural properties may be important for topical care products with a Sun Protection Factor and skincare products. For example, the film-forming property of the formulation affects the Sun Protection Factor that is provided by the product when applied to the skin. The textural or skin feel properties may be important for consumer acceptance of the product. Furthermore, it is desirable that suitable formulations should allow for inclusion of other skin care actives in the formulations.

The use of soft gelatin and Vegicaps® capsules to deliver topical formulations including UV ingredients would provide an attractive, convenient single use option for dispensing fill compositions containing UV ingredients potentially providing an SPF rating. Typically, these capsules contain a unit dose of from 0.09 ml to 4.5 ml of fill material and have a "twist-off" or other removable feature at one end for dispensing the fill material. Such Soft gelatin and Vegicaps® capsules can be prepared by methods well known for the preparation of softgels for oral dosage formulations, i.e. by encapsulating the fill material between two sheets of gelatin as it passes between a pair of die rolls having surface cavities shaped to form the desired shape of the resulting softgel.

It is also desirable to provide formulations that are compatibile with standard and other encapsulation technologies and capsule forms including Jintan™ and liquid filled two-piece hard shell capsule technologies.

The present invention provides formulations allowing inclusion of UV ingredients that support a SPF, and provides compatibility with different encapsulation technologies and capsule forms.

### SUMMARY OF THE INVENTION

In one aspect, the present invention provides an anhydrous fill composition for a topical product. The fill composition includes a branched hydrocarbon base oil having 9-16 carbon atoms per molecule; a sufficient amount of a viscosity adjusting agent comprising 4.0 to 16 wt% of at least one silicone elastomer in combination with 0.1 to 7 wt % of at least one synthetic wax and 0.1 to 2 wt% of at least one clay-based viscosity adjusting agent, wherein the weight percentages are based on the total weight of the anhydrous fill composition; and one or more UV filters.

In a second aspect, the present invention provides a storage stable anhydrous fill formulation compatible with a variety of different capsule technologies and which comprises the anhydrous fill composition and optionally one or more UV defense boosters.

In a third aspect, this invention provides a soft gel capsule containing comprising a soft gelatin capsule shell and the anhydrous fill composition within the shell, which, when dispensed, provides a film including one or more sun protection ingredients. The fill material may optionally include one or more UV defense boosters.

### BRIEF DESCRIPTION OF THE DRAWINGS

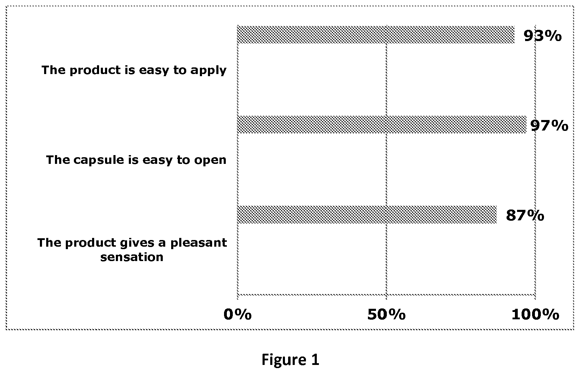

Figure 1 shows the results of a subjective assessment of the short term-effects of the product of Example 1.

### DETAILED DESCRIPTION OF THE PREFERRED EMBODIMENT(S)

It must be noted that as used herein and in the appended claims, the singular forms "a", "an", and "the" include plural references unless the context clearly dictates otherwise. Furthermore, the terms "a" (or "an"), "one or more" and "at least one" can be used interchangeably herein. The terms "comprising", "including", "having" and "constructed from" can also be used interchangeably.

As used herein, "capsule" refers to a two piece hard shell or a soft gelatin and/or non-gelatin capsule manufactured utilising a rotary die machine.

As used herein, "unit dose" refers to a dose of from 0.09 ml to 4.5ml.

In one embodiment, the invention relates to an anhydrous fill composition for a topical product. The fill composition includes a branched hydrocarbon base oil having 9-16 carbon atoms per molecule; a sufficient amount of a viscosity adjusting agent comprising 4.0 to 16 wt% of at least one silicone elastomer in combination with 0.1 to 7 wt% of at least one synthetic wax and 0.1 to 2 wt% of at least one clay-based viscosity adjusting agent, wherein the weight percentages are based on the total weight of the anhydrous fill composition; and one or more UV filters. The anhydrous fill composition may optionally include one or more UV defense boosters. This anhydrous fill composition can be used for a wide range of formulations including formulations containing skin care components and formulations designed for use a various types of antiaging products.

In another embodiment, the invention relates to the anhydrous fill composition adapted for single-use application using a single-use container. The fill composition contains one or more UV ingredients. The fill composition is storage stable, capable of film formation upon application and is compatible with a variety of different single-use container technologies including softgel capsules and hard shell capsules.

In another embodiment, the invention relates to a softgel capsule containing the anhydrous fill composition. Formulations according to this invention are stable and provide cosmetically acceptable topical composition and do not destabilize softgel capsules including, but not limited to, Softgel or Vegicaps® capsules, as well as being compatible with pearls and drops, Thus, the present invention provides attractive topical formulations for UV filters or suncreens, optionally including one or more UV defense boosters in convenient single-use containers. The single dose formulations of the present invention can also be used in other anhydrous fill/oily fill compatible systems, such as, for example, two-piece hard shell capsules, Jintan capsules, animal and non-gelatin based capsule shell technologies and Vegicaps® capsule formulations.

The compatibility of the fill formulation with both UV ingredients and capsule technologies leads to the ability to provide a composition in unit dose form that is capable of forming a homogenous film on the skin. As a result,the composition may be used as a vehicle for a sun protection factor (SPF), as well as being able to deliver other desirable products features for the skin. The texture of the formulation in the invention permits inclusion of UV ingredients including at least UV filters and optionally one or more UV defense boosters which, in turn, provide a SPF usable in a wide range of skin care formulations including those delivered by a unit dose delivery form such as capsules.

The base composition may include a variety of ingredients. In one embodiment, the base composition includes a thickened hydrocarbon base adapted for supporting one or more UV filters, and optionally one or more UV defense boosters.The thickened hydrocarbon base can be used to form the fill composition that is in the form of an anhydrous topical product.

The base composition may contain one or more light partially volatile oils. Suitable oils are hydrocarbons containing only carbon and hydrogen. In one embodiment, the hydrocarbon oil may be a branched chain aliphatic hydrocarbon having 9 to 16 carbon atoms. One example of a hydrocarbon oil is isododecane.

Another class of volatile oils useful for the present invention include low viscosity linear siloxanes such as, for example, disiloxane, trisiloxane and dimethicone.. The volatile oils may, in some embodiments, function as a solvent for the viscosity control agents described below.

The viscosity of the hydrocarbon oil is adjusted using one or more suitable viscosity control agents. The hydrocarbon oil component is thickened, or gelled, to increase its viscosity using one or more viscosity control agents that are thickening agents either taken alone or in combination. Suitable viscosity control agents include, for example, silica, fumed silica and its reaction products such a silica silylate, silica dimethyl silylate and silica dimethicone silylate, and other anhydrous viscosity control agents. For example, viscosity control agents obtained by the reaction of a quaternary ammonium salt and hectorite or bentonite, such as disteardimonium hectorite, stearalkonium bentonite and stearalkonium hectorite. Hectorite is one of the montmorillonite minerals that are the principal constituents of bentonite clay. Bentonite is a native hydrated colloidal aluminum silicate clay. Other suitable viscosity control agents include, for example, carbonates such as propylene carbonate and dicapryl carbonate, cross-linked siloxane polymers, polydimethylsiloxanes, and synthetic waxes such as a polyethylene polymer, which is predominantly made up of polymer units having at least 30 or more carbon atoms. The polyethylenes may have a melting point range of 50°-120°C.

A combination of two or more viscosity control agents may be employed to provide a balance between viscosity, stability, skin feel, film-forming ability and the ability to encapsulate the material. One class of viscosity modifiers useful for this purpose are silicone elastomers. The silicone elastomers is used in combination with other classes of viscosity modifying agents. In the invention as claimed, a combination of one or more silicone elastomers with one or more synthetic waxes and one or more clay-based viscosity adjussting agents is employed. Each of the foregoing combinations may be further combined with one or more silica-based viscosity control agents.

Silicone elastomer viscosity control agents include, for example, cross-linked siloxane polymers and polydimethylsiloxanes. More specific examples include INCI polysilicone-11, a crosslinked dimethyl siloxane formed by the reaction of bis-vinyldimethicone and hydrogen dimethicone in the presence of a solvent. Bis-vinyldimethicone is a derivative of dimethicone wherein one methyl group at each end of the siloxane chain has been replaced with a vinyl group. Hydrogen dimethicone is a derivative of dimethicone wherein some of the methyl groups have been replaced with a hydrogen atom. INCI stands for International Nomenclature for Cosmetic Ingredients.

Another suitable cross-linked siloxane polymer is INCI vinyldimethyl/trimethylsiloxysilicate/dimethicone crosspolymer which is formed by crosslinking hydrogen dimethicone with vinyldimethyl/trimethylsiloxysilicate.

Another suitable cross-linked siloxane polymer is INCI dimethicone/vinyl dimethicone crosspolymer which is a crosslinked dimethyl siloxane polymer formed by the reaction of hydrogen dimethicone and vinyl dimethicone. Vinyl dimethicone is a derivative of dimethicone where some of the methyl groups have been replaced with vinyl groups. The vinyl groups can be present at the ends of the siloxane chain or pendant to the siloxane chain.

Another suitable cross-linked siloxane polymer is INCI dimethicone crosspolymer, which is a polymer of dimethicone crosslinked with an alkyl group and is available as Dow Corning EL 8040ID. Mixtures of two or more of these silicone elastomers may be used alone or in combination with other viscosity control agents.

The amount of silicone elastomer viscosity control agent employed in the fill composition is from 4.0 to 16.0 wt%, and, more preferably from 5 to about14 wt%.

Synthetic wax viscosity control agents, include, for example, polyethylene polymers, which are predominantly made up of polymer units having at least 30 or more carbon atoms. The preferred polythylene wax may include low levels such as between 0.1 and 10%, or, more preferably between 0.5 -5 % of polymer units having 26 or more carbon atoms with a melting point range of 60°- 80°C. The polyethylenes are preferred since they thicken, are film-forming and can improve the skin feel of the product. Mixtures of two or more of these synthetic wax materials may be used alone or in combination with other viscosity control agents. The amount of synthetic wax viscosity control agent employed in the fill composition is from 0.1 to 7 wt.%, and, more preferably from 0.5 to 5 wt%.

Clay-based viscosity control agents include, for example, viscosity control agents obtained by the reaction of a quaternary ammonium salt and hectorite or bentonite, such as disteardimonium hectorite, stearalkonium bentonite and stearalkonium hectorite. Hectorite is one of the montmorillonite minerals that are the principal constituents of bentonite clay. Bentonite is a native hydrated colloidal aluminum silicate clay. Mixtures of two or more of these materials may be used alone or in combination with other viscosity control agents. The amount of the clay-based viscosity control agent employed in the fill composition is from 0.1 to 2 wt%, and, more preferably from 0.15 to 1 wt%.

Silica-based viscosity control agents include, for example, silica, fumed silica and its reaction products such a silica silylate, silica dimethyl silylate and silica dimethicone silylate, and other anhydrous viscosity control agents. Mixtures of two or more of these materials may be used alone or in combination with other viscosity control agents. The amount of the silica-based viscosity control agent employed in the fill composition may be from 0.01 to 5 wt%, and, more preferably from 0.02 to 3.5 wt%.

One particularly suitable base oil composition is a thickened hydrocarbon oil composition, such as isododecane thickened with a cross-linked siloxane polymer such as INCI polysilicone-11, INCI vinyldimethyl/trimethylsiloxysilicate/dimethicone crosspolymer, INCI dimethicone/vinyl dimethicone crosspolymer and INCI dimethicone crosspolymer.

The invention also relates to fill formulations useful as cosmetic agents, skincare products, over the counter (OTC) topical products, topical medical products, pharmaceutical products as well as other types of topical products. The fill formulations of the present invention comprise the base composition described above and a UV component such as a UV filter, and optionally one or more UV defense boosters. The fill formulations may optionally further comprise one or more additional UV filters. Exemplary fill formulations in accordance with the invention may include, for example, the ingredients set forth in Table 1.

**Table 1**

| **Component** | **Wt.%** | **Preferrred Wt%** | **More Preferrred Wt.%** |
|---|---|---|---|
| Thickening agent(s) and/or Viscosity control agent(s) | 5-70 | 8-40 | 10-20 |
| Hydrocarbon oil(s) | 20-70 | 30-65 | 40-60 |
| UV filter(s) | 7-30 | 8-20 | 8-15 |
| UV stabilizer(s) | 0,0-10 | 0.5-10 | 3-8 |
| Skin conditioning agent(s) | 0-20 | 5-20 | 10-20 |

A UV filter is an ingredient that absorbs, reflects, or scatters radiation in the UV wavelength range, specifically at wavelengths of from 290 to 400 nanometers. The fill composition of the present invention may include one or more UV filters. One type of UV filter that may be employed in the present invention are UV filter substances based on triazine derivatives. One class of UV filters that may be used in the present invention include alkyl methoxy cinnamates such as ethyl hexyl methoxy cinnamate, octyl methoxy cinnamate, and isoamyl methoxy cinnamate. Other UV filters that may be used in the present invention include oxybenzone, homosalate, octocrylene, octyl salicylate, avobenzone, titanium dioxide, and zinc oxide. Diethylamino hydroxybenzoyl hexyl benzoate may also be used as a UV filter substance.

UV defense boosters are ingredients such as ingredients that improve formulation efficiency, photostabilizers, film-forming agents, antioxidants, and ingredients which improve solvency of the UV filters. The fill formulations may optionally include one or more of each of these UV boosters as well as mixtures of these UV boosters.

Photostabilizers are employed in cosmetics to protect the product from chemical or physical deterioration induced by light. In many products, a combination of UV filters and photostabilizers is utilized to protect the skin or hair from ultraviolet light. The fill compositions of the present invention may include photostabilizers or a combination of one or more UV filters and one or more photostabilizers. Suitable photostabilizers may include, for example, diethylamino hydroxybenzoyl hexyl benzoate, ethylhexyl methoxycrylene and diethylhexyl syringylidene malonate.

One particularly useful combination of a UV filter and a photostabilizer is ethylhexyl methoxycinnamate in combination with diethylamino hydroxybenzoyl hexyl benzoate. This combination, alone or in combination with other photostabilizers can provide an SPF of 15 or an SPF of more than 20, as measured by *in vivo* determination using the method of ISO24444 and a UVA protection factor (PF) of 7.2, as measured by *in vitro* determination using the method of ISO24443.

The final viscosity range of the fill composition is from 10 Pa·s (10,000 cP (centipoise)) to 60 Pa·s (60,000 cP), or from 20 Pa·s (20,000 cP) to 50 Pa·s (50,000 cP).

The fill formulation may include other optional ingredients such as, for example, compounds that have a photo-protective effect, skin brighteners, skin conditioners to improve appearance, restore suppleness and protect the skin, skin smoothing ingredients, skin soothing agents, moisturizers, melanin inhibitors, free radical quenchers, anti-inflammatories and mixtures thereof. These additional materials may be employed in conventional amounts known to skilled formulators of products employing these ingredients.

The fill formulations of the present invention may be employed for daily use in the form of a skin care product that helps to protect the skin from sun damage and may, optionally, also help to prevent premature skin-aging. These products may be conveniently offered in softgel or Vegicaps® capsules in unit-dosages, for on-the-go application. The fill formulation is formulated such that when it is dispensed, it forms a film on the skin that is substantially homogeneous since this can improve the SPF of the product upon application.

Thus, the formulations of the present invention can be employed to provide sun protection, e.g. to provide an SPF of at least 15 or an SPF of at least 20. One or more UV filters and optionally one or more UV defense boosters can be employed to provide SPF 15 or 20, if desired.

Anti-ageing, skincare, pharmaceutical or cosmetic formulations in accordance with the present invention can be formulated to smooth wrinkles, particularly in the short term, to brighten skin, to help reduce and prevent melanic dark spots, and/or to provide free-radical protection . In one embodiment, the invention provides a product for daily use that is formulated to combine a short term wrinkle smoothing effect and sun protection with a long term skin brightening effect.

In a further aspect, the present invention relates to a capsule containing a fill formulation as described above. The capsules may be, for example, softgel or Vegicaps® capsules. The anhydrous fill composition may also be used in a hard shell capsule. In some embodiments, the capsules comprise gelatin and in other embodiments the capsules do not employ gelatin in the capsule composition. In one embodiment, the capsule is sized to provide a unit-dosage of the fill formulation.

Ingredient names are taken from the wINCI Web Based International Cosmetic Ingredient Dictionary & Handbook and the CosIng cosmetic ingredients database.

### Example 1

The formulation of Table 2 was employed to provide a sun protection factor >/= 15 for a daily skin care product.The primary ingredients of the formulation are set forth in Table 2 below with the amounts given in parts per hundred by weight, based on the total weight of the formulation. The formulation included isododecane as the base oil and polyethylene, silica , dimethicone crosspolymer, stearalkonium hectorite, propylene carbonate and dicapryl carbonate as viscosity control agents. The formulation included an ethylhexyl methoxycinnamate UV filter as well as diethylamino hydroxybenzoyl hexyl benzoate, ethylhexyl methoxycrylene and diethylhexyl syringylidenemalonate for the purpose of protecting the skin from sun damage and to help prevent photo-ageing.

**Table 2**

| **Ingredient** | **pph** |
|---|---|
| ISODODECANE | 50.2600 |
| DIMETHICONE CROSSPOLYMER | 7.0400 |
| ETHYLHEXYL METHOXYCINNAMATE | 7.0000 |
| NEOPENTYL GLYCOL DIHEPTANOATE | 5.7000 |
| SILICA | 4.0000 |
| ETHYLHEXYL METHOXYCRYLENE | 4.0000 |
| POLYETHYLENE | 4.0000 |
| C12-15 ALKYL BENZOATE | 4.0000 |
| DIETHYLAMINO HYDROXYBENZOYL HEXYL BENZOATE | 3.5000 |
| CAPRYLIC/CAPRIC TRIGLYCERIDE | 3.1946 |
| DICAPRYLYL CARBONATE | 2.6100 |
| DIETHYLHEXYL SYRINGYLIDENEMALONATE | 1.8000 |
| ETHYLHEXYL PALMITATE | 0.9530 |
| STEARALKONIUM HECTORITE | 0.3000 |
| PROPYLENE CARBONATE | 0.0900 |
| SILICA DIMETHYL SILYLATE | 0.0250 |

The composition of Table 2 was tested for UV protection factor and the results are given in Table 3 below.

**Table 3**

| | |
|---|---|
| **SPF** - **UVB Protection** | 24.2 |
| In vivo determination-ISO24444 | |
| **UVA PF (Protection Factor)** | 7.2 |
| In vitro determination - ISO24443 | |
| In vitro Critical wavelength evaluation with pre-irradiation COLIPA 2011 method : 372 nm | |

A subjective assessment of the product of Table 2 was carried out by providing the product to 30 volunteer test subjects ages 18 to 55. One daily application of the product on the fact was carried out by the test subjects at home under normal conditions of use. At least two hours after the first application, the volunteers provided a subjective assessment of the product. The results of the subjective assessment are shown in Figure 1. The subjective assessment of Figure 1 shows that the product was well received by the test volunteers.

## Claims

1. An anhydrous fill composition for a topical product, said fill composition comprising:
(a) a branched chain hydrocarbon base oil having 9-16 carbon atoms per molecule;
(b) a sufficient amount of a viscosity adjusting agent comprising 4.0 to 16 wt% of at least one silicone elastomer in combination with 0.1 to 7 wt% of at least one synthetic wax and 0.1 to 2 wt% of at least one clay-based viscosity adjusting agent, wherein the weight percentages are based on the total weight of the anhydrous fill composition; and
(c) one or more UV filters.

2. The anhydrous fill composition as claimed in claim 1, wherein said base oil is isododecane and said at least one silicone elastomer viscosity adjusting agent is a cross-linked siloxane polymer.

3. The anhydrous fill composition as claimed in claim 1, wherein said base oil is isododecane.

4. The anhydrous fill composition as claimed in any one of claims 1-3, wherein said one or more UV filters comprises an alkyl methoxy cinnamate.

5. The anhydrous fill composition as claimed in any one of claims 1-3, wherein said one or more UV filters is selected from the group consisting of ethyl hexyl methoxy cinnamate, octyl methoxy cinnamate, isoamyl methoxy cinnamate and mixtures thereof.

6. The anhydrous fill composition as claimed in any one of claims 1-3, wherein said one or more UV filters is selected from the group consisting of ethyl hexyl methoxy cinnamate, octyl methoxy cinnamate, isoamyl methoxy cinnamate, oxybenzone, homosalate, octocrylene, octyl salicylate, avobenzone, titanium dioxide, zinc oxide and mixtures thereof.

7. The anhydrous fill composition as claimed in claim 1, further comprising one or more photostabilizers selected from diethylamino hydroxybenzoyl hexyl benzoate, ethylhexyl methoxycrylene, and diethylhexyl syringylidene malonate.

8. The anhydrous fill composition as claimed in any one of claims 1-7, further comprising a UV defense booster selected from film-forming agents and antioxidants.

9. The anhydrous fill composition as claimed in any one of claims 1-8, wherein said clay-based viscosity adjusting agent is a product of a reaction of a quaternary ammonium salt and hectorite or bentonite.

10. The anhydrous fill composition as claimed in any one of claims 1-8, wherein said viscosity adjusting agent further comprises at least one silica-based viscosity adjusting agent.

11. The anhydrous fill composition as claimed in any one of claims 1-8 and 10, wherein said silicone elastomer viscosity adjusting agent is selected from the group consisting of INCI polysilicone-11, INCI vinyldimethyl/trimethylsiloxysilicate/dimethicone crosspolymer, INCI dimethicone/vinyl dimethicone crosspolymer and INCI dimethicone crosspolymer.

12. The anhydrous fill composition as claimed in claim 10, wherein the silica-based viscosity adjusting agent is present in an amount of from 0.01 wt.% to 5 wt.%, based on the total weight of the anhydrous fill composition.

13. The anhydrous fill composition as claimed in any one of claims 1-12, wherein:
the hydrocarbon base oil is present in an amount of from 20 wt.% to 70 wt.%, the thickening agent(s) and/or the viscosity adjusting agent is present in an amount of from 5 wt.% to 70 wt.%, the one or more UV filters is present in an amount of from 7 wt.% to 30 wt.%, a UV stabilizer is present in an amount of from 0.0 wt.% to 10 wt.%, and a skin condition agent(s) is present in an amount of from 0 wt.% to 20 wt.%; or
the hydrocarbon base oil is present in an amount of from 30 wt.% to 65 wt.%,the thickening agent(s) and/or the viscosity adjusting agent is present in an amount of from 8 wt.% to 40 wt.%, the one or more UV filters is present in an amount of from 8 to 20 wt.%, the UV stabilizer is present in an amount of from 0.5 wt.% to 10 wt.%, and the skin condition agent(s) is present in an amount of from 5 wt.% to 20 wt.%, or
the hydrocarbon base oil is present in an amount of from 40 wt.% to 60 wt.%, the thickening agent(s) and/or the viscosity adjusting agent is present in an amount of from 10 wt.% to 20 wt.%, the one or more UV filters is present in an amount of from 8 wt.% to 15 wt.%, the UV stabilizer is present in an amount of from 3 wt.% to 8 wt.%, and the skin conditioning agent(s) is present in an amount of from 10 wt.% to 20 wt.%, based on the total weight of the anhydrous fill composition.

14. A soft gel capsule containing a fill composition as claimed in any one of claims 1-13.

## Patentansprüche

1. Eine wasserfreie Füllzusammensetzung für ein topisches Produkt, wobei die Füllzusammensetzung Folgendes umfasst:
(a) ein verzweigtkettiges Kohlenwasserstoff-Basisöl mit 9 bis 16 Kohlenstoffatomen pro Molekül,
(b) eine ausreichende Menge eines viskositätsregulierenden Mittels, umfassend 4,0 bis 16 % Massenanteil mindestens eines Silikonelastomers verbunden mit 0,1 bis 7 % Massenanteil mindestens eines synthetischen Wachses und 0,1 bis 2 % Massenanteil mindestens eines viskositätsregulierenden Mittels auf Tonbasis, wobei sich die Massenprozentsätze auf die Gesamtmasse der wasserfreien Füllzusammensetzung beziehen, und
(c) einen oder mehrere UV-Filter.

2. Wasserfreie Füllzusammensetzung nach Anspruch 1, wobei das Basisöl Isododecan ist und das mindestens eine viskositätsregulierende Mittel aus Silikonelastomer ein vernetztes Siloxanpolymer ist.

3. Wasserfreie Füllzusammensetzung nach Anspruch 1, wobei das Basisöl Isododecan ist.

4. Wasserfreie Füllzusammensetzung nach einem der Ansprüche 1 bis 3, wobei der eine oder die mehreren UV-Filter ein Alkylmethoxycinnamat enthalten.

5. Wasserfreie Füllzusammensetzung nach einem der Ansprüche 1 bis 3, wobei der eine oder die mehreren UV-Filter ausgewählt sind aus der Gruppe bestehend aus Ethylhexylmethoxycinnamat, Octylmethoxycinnamat, Isoamylmethoxycinnamat und Gemischen davon.

6. Wasserfreie Füllzusammensetzung nach einem der Ansprüche 1 bis 3, wobei der eine oder die mehreren UV-Filter ausgewählt sind aus der Gruppe bestehend aus Ethylhexylmethoxycinnamat, Octylmethoxycinnamat, Isoamylmethoxycinnamat, Oxybenzon, Homosalat, Octocrilen, Octylsalicylat, Avobenzon, Titandioxid, Zinkoxid und Gemischen davon.

7. Wasserfreie Füllzusammensetzung nach Anspruch 1, ferner umfassend einen oder mehrere Photostabilisatoren, ausgewählt aus Diethylaminohydroxybenzoylhexylbenzoat, Ethylhexylmethoxycrylen und Diethylhexylsyringylidenmalonat.

8. Wasserfreie Füllzusammensetzung nach einem der Ansprüche 1 bis 7, ferner umfassend einen aus filmbildenden Mitteln und Antioxidantien ausgewählten UV-Abwehrverstärker.

9. Wasserfreie Füllzusammensetzung nach einem der Ansprüche 1 bis 8, wobei das viskositätsregulierende Mittel auf Tonbasis ein Produkt einer Reaktion eines quaternären Ammoniumsalzes und Hectorit oder Bentonit ist.

10. Wasserfreie Füllzusammensetzung nach einem der Ansprüche 1 bis 8, wobei das viskositätsregulierende Mittel ferner mindestens ein viskositätsregulierendes Mittel auf Siliciumdioxidbasis umfasst.

11. Wasserfreie Füllzusammensetzung nach einem der Ansprüche 1 bis 8 und 10, wobei das viskositätsregulierende Mittel aus Silikonelastomer ausgewählt ist aus der Gruppe bestehend aus INCI-Polysilikon-11, INCI-Vinyldimethyl/Trimethylsiloxysilikat/Dimethicon-Kreuzpolymer, INCI-Dimethicon/Vinyldimethicon-Kreuzpolymer und INCI-Dimethicon-Kreuzpolymer.

12. Wasserfreie Füllzusammensetzung nach Anspruch 10, wobei das viskositätsregulierende Mittel auf Siliciumdioxidbasis in einer Menge von 0,01 % Massenanteil bis 5 % Massenanteil, bezogen auf die Gesamtmasse der wasserfreien Füllzusammensetzung, vorhanden ist.

13. Wasserfreie Füllzusammensetzung nach einem der Ansprüche 1 bis 12, wobei: das Kohlenwasserstoff-Basisöl in einer Menge von 20 % Massenanteil bis 70 % Massenanteil vorhanden ist, das (die) Verdickungsmittel und/oder das viskositätsregulierende Mittel in einer Menge von 5 % Massenanteil bis 70 % Massenanteil vorhanden ist (sind), der eine oder die mehreren UV-Filter in einer Menge von 7 % Massenanteil bis 30 % Massenanteil vorhanden ist (sind), ein UV-Stabilisator in einer Menge von 0,0 % Massenanteil bis 10 % Massenanteil vorhanden ist und (ein) Hautpflegemittel in einer Menge von 0 % Massenanteil bis 20 % Massenanteil vorhanden ist (sind), oder
das Kohlenwasserstoff-Basisöl in einer Menge von 30 % Massenanteil bis 65 % Massenanteil vorhanden ist, das (die) Verdickungsmittel und/oder das viskositätsregulierende Mittel in einer Menge von 8 % Massenanteil bis 40 % Massenanteil vorhanden ist (sind), der (die) eine oder mehreren UV-Filter in einer Menge von 8 bis 20 % Massenanteil vorhanden ist (sind), der UV-Stabilisator in einer Menge von 0,5 % Massenanteil bis 10 % Massenanteil vorhanden ist und das (die) Hautpflegemittel in einer Menge von 5 % Massenanteil bis 20 % Massenanteil vorhanden ist (sind), oder
das Kohlenwasserstoff-Basisöl in einer Menge von 40 % Massenanteil bis 60 % Massenanteil vorhanden ist, das (die) Verdickungsmittel und/oder das viskositätsregulierende Mittel in einer Menge von 10 % Massenanteil bis 20 % Massenanteil vorhanden ist (sind), der eine oder die mehreren UV-Filter in einer Menge von 8 % Massenanteil bis 15 % Massenanteil vorhanden ist (sind), der UV-Stabilisator in einer Menge von 3 % Massenanteil bis 8 % Massenanteil vorhanden ist und das (die) Hautpflegemittel in einer Menge von 10 % Massenanteil bis 20 % Massenanteil vorhanden ist (sind), bezogen auf die Gesamtmasse der wasserfreien Füllzusammensetzung.

14. Weichgelkapsel, die eine Füllzusammensetzung nach einem der Ansprüche 1 bis 13 enthält.

## Revendications

1. Composition de remplissage anhydre pour produit topique, ladite composition de remplissage comprenant :
(a) une huile de base hydrocarbonée à chaîne ramifiée ayant de 9 à 16 atomes de carbone par molécule ;
(b) une quantité suffisante d'un agent d'ajustement de la viscosité comprenant de 4,0 à 16 % en poids d'au moins un élastomère à la silicone associé avec 0,1 à 7 % en poids d'au moins une cire synthétique et 0,1 à 2 % en poids d'au moins un agent d'ajustement de la viscosité à base d'argile, les pourcentages en poids étant basés sur le poids total de la composition de remplissage anhydre ; et
(c) un ou plusieurs filtres anti-UV.

2. Composition de remplissage anhydre comme revendiquée dans la revendication 1, ladite huile de base étant de l'isododécane et ledit au moins un agent d'ajustement de la viscosité élastomère à la silicone étant un polymère de siloxane réticulé.

3. Composition de remplissage anhydre comme revendiquée dans la revendication 1, ladite huile de base étant de l'isododécane.

4. Composition de remplissage anhydre comme revendiquée dans l'une quelconque des revendications 1 à 3, lesdits un ou plusieurs filtres anti-UV comprenant un méthoxycinnamate d'alkyle.

5. Composition de remplissage anhydre comme revendiquée dans l'une quelconque des revendications 1 à 3, lesdits un ou plusieurs filtres anti-UV étant choisis dans le groupe constitué par le méthoxycinnamate d'éthylhexyle, le méthoxycinnamate d'octyle, le méthoxycinnamate d'isoamyle et leurs mélanges.

6. Composition de remplissage anhydre comme revendiquée dans l'une quelconque des revendications 1 à 3, lesdits un ou plusieurs filtres anti-UV étant choisis dans le groupe constitué par le méthoxycinnamate d'éthylhexyle, le méthoxycinnamate d'octyle, le méthoxycinnamate d'isoamyle, l'oxybenzone, l'homosalate, l'octocrylène, le salicylate d'octyle, l'avobenzone, le dioxyde de titane, l'oxyde de zinc et leurs mélanges.

7. Composition de remplissage anhydre comme revendiquée dans la revendication 1, comprenant en outre un ou plusieurs agents photostabilisants choisis parmi le diéthylaminohydroxybenzoylbenzoate d'hexyle, l'éthylhexylméthoxycrylène et le syringylidènemalonate de di(éthylhexyle).

8. Composition de remplissage anhydre comme revendiquée dans l'une quelconque des revendications 1 à 7, comprenant en outre un renforçateur de défense anti-UV choisi parmi des agents filmogènes et des antioxydants.

9. Composition de remplissage anhydre comme revendiquée dans l'une quelconque des revendications 1 à 8, ledit agent d'ajustement de la viscosité à base d'argile étant un produit de la réaction entre un sel d'ammonium quaternaire et de l'hectorite ou de la bentonite.

10. Composition de remplissage anhydre comme revendiquée dans l'une quelconque des revendications 1 à 8, ledit agent d'ajustement de la viscosité comprenant en outre au moins un agent d'ajustement de la viscosité à base de silice.

11. Composition de remplissage anhydre comme revendiquée dans l'une quelconque des revendications 1 à 8 et 10, ledit agent d'ajustement de la viscosité élastomère à la silicone étant choisi dans le groupe constitué par l'ingrédient INCI polysilicone-11, l'ingrédient INCI vinyldimethyl/trimethylsiloxysilicate/dimethicone crosspolymer, l'ingrédient INCI dimethicone/vinyldimethicone crosspolymer et l'ingrédient INCI dimethicone crosspolymer.

12. Composition de remplissage anhydre comme revendiquée dans la revendication 10, ledit agent d'ajustement de la viscosité à base de silice étant présent à raison de 0,01 % en poids à 5 % en poids, sur la base du poids total de la composition de remplissage anhydre.

13. Composition de remplissage anhydre comme revendiquée dans l'une quelconque des revendications 1 à 12, dans laquelle :
l'huile de base hydrocarbonée est présente à hauteur de 20 % en poids à 70 % en poids, le ou les agents épaississants et/ou l'agent d'ajustement de la viscosité sont présents à raison de 5 % en poids à 70 % en poids, le ou les filtres anti-UV sont présents à raison de 7 % en poids à 30 % en poids, un stabilisant UV est présent à raison de 0,0 % en poids à 10 % en poids et un ou plusieurs agents de conditionnement de la peau sont présents à raison de 0 % en poids à 20 % en poids ; ou
l'huile de base hydrocarbonée est présente à hauteur de 30 % en poids à 65 % en poids, le ou les agents épaississants et/ou l'agent d'ajustement de la viscosité sont présents à raison de 8 % en poids à 40 % en poids, le ou les filtres anti-UV sont présents à raison de 8 % en poids à 20 % en poids, le stabilisant UV est présent à raison de 0,5 % en poids à 10 % en poids et le ou les agents de conditionnement de la peau sont présents à raison de 5 % en poids à 20 % en poids, ou
l'huile de base hydrocarbonée est présente à hauteur de 40 % en poids à 60 % en poids, le ou les agents épaississants et/ou l'agent d'ajustement de la viscosité sont présents à raison de 10 % en poids à 20 % en poids, le ou les filtres anti-UV sont présents à raison de 8 % en poids à 15 % en poids, le stabilisant UV est présent à raison de 3 % en poids à 8 % en poids et le ou les agents de conditionnement de la peau sont présents à raison de 10 % en poids à 20 % en poids, sur la base du poids total de la composition de remplissage anhydre.

14. Capsule molle comprenant une composition de remplissage ainsi que revendiqué dans l'une quelconque des revendications 1 à 13.
